# EUROPEAN PATENT SPECIFICATION

(11) **EP 0 494 146 B1**
(45) Date of publication and mention of the grant of the patent: **25.10.1995**
(21) Application number: 90910860.7
(22) Date of filing: 27.07.1990
(51) Int. Cl.: C07C 61/40, C07C 51/60, C07C 69/743

(54) **PROCESS FOR THE PREPARATION OF OPTICALLY ACTIVE OR RACEMIC CYCLOPROPANE-CARBOXYLIC-ACID CHLORIDES**
VERFAHREN ZUR HERSTELLUNG VON OPTISCH AKTIVEN ODER RACEMISCHEN CYCLOPROPANCARBONSÄURECHLORIDEN
PROCEDE DE PREPARATION DE CHLORURES D'ACIDE CARBOXYLIQUE-CYCLOPROPANE RACEMIQUES OU OPTIQUEMENT ACTIFS

(43) Date of publication of application: 15.07.1992
(73) Proprietor: CHINOIN Gyògyszer és Vegyészeti Termékek Gyára RT., H-1045 Budapest IV (HU)
(72) Inventor: SZEKELY, István, H-2120 Dunakeszi (HU); ZOLTAN, Sándor, H-1142 Budapest (HU); NAGY, Lajos, H-2000 Szentendre (HU); BERTOK, Béla, H-1222 Budapest (HU); SOMFAI, Eva, H-1014 Budapest (HU); HIDASL, György, H-1142 Budapest (HU); IMREI, Lajos, H-1131 Budapest (HU); LAK, István, H-1016 Budapest (HU); THURNER, Angelika, H-1092 Budapest (HU)
(74) Representative: Skailes, Humphrey John
(86) International application number: HU9000054
(87) International publication number: WO9202482

(56) References cited:
- DE-A-13 023 484
- DE-A-13 445 504

## Description

The present invention relates to a new process for the preparation of cyclopropane carboxylic acid chlorides, which are intermediates for pyrethroid synthesis.

The synthetic pyrethroids are widely used insecticides. Several processes are known for their preparation (e.g. HU-A 55/85 and HU-B 197,292). In the most common industrial processes the cyclopropane carboxylic acid derivatives are esterified - taking in consideration the sensitivity of these compounds - under very mild conditions by the activation of the alcohol and the acid components respectively. Of these methods, the use of cyclopropane carboxylic acid chlorides is the most common (e.g. HU-B 170,866).

Most of the known methods for the preparation of carbonic acid chlorides in question have many disadvantages. The application of these processes is very much limited by the above mentioned sensitivity and the inclination to isomerization and decomposition of the compounds. For example, in the conventional preparation of cis-permethrin-acid chloride with thionyl chloride, even under the most mild conditions a 5-10% cis-trans isomerisation can be observed, which is a great disadvantage because of the great difference in the biological efficacy of the individual isomers (GB 1,540,632, Pestic. Sci. (1978), 9, 112-16; Pestic Sci., (1980), 11, 169-179, EP-A 067,461).

The product thus obtained can be used only infrequently without further purification because of the decomposition products and traces of acid present. This is generally true of the cyclopropane carboxylic acids. For the preparation of pyrethroids of good quality, of the methods for consideration industrially, only fine vacuum distillation can be used for purification. On the other hand, because of the high sensitivity of the product, the highly acidic distillation conditions cause great losses.

In the case of optically active compounds the decomposition is unambiguously demonstrated and can be explained by the catalytic effect of the strong acids present of formed the reaction. This is general for cyclopropane carboxylic acids (Tetrahedron Letters, Vol. 25, No. 15), 1595-1598, 1984).

An improvement was the use, as an activated derivative of cyclopropane carboxylic acid, of a dimethyl-(acyloxy-methylenediene)-ammonium salt of the formula (IV) 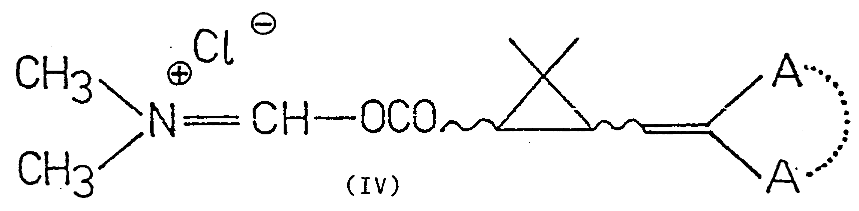
(HU-B 444). According to the examples, the dimethylformamide was reacted in anhydrous acetonitrile or toluene at temperatures between -20°C and 0°C with oxalyl chloride or phosgene and the suspension obtained was further reacted at -15°C with the cyclopropane carboxylic acid. To the mixture thus obtained suitable alcohol components were added at -20°C. This method gave in a very simple way tetramethrin, permethrin or cinnerin as products without decomposition or isomerisation of the activated acid derivative. The activated acid derivative cannot be stored because in the presence of certain (primarily metallic) contaminations it can undergo to self catalyzed decomposition, which can cause an explosion even at room temperature. As the waste acid formed from the reagent in the activation is always present as a complex, the reaction mixture is not harmless. A further disadvantage is that, according to the patent examples, in the esterification step a large excess of organic base (e.g. pyridine) is needed to neutralize the waste acid. This increases the costs and the processing is made more difficult. In the direct use of the reaction mixture in the esterification, because of the lack of the purifying step, the contaminations and by-products can get into the pyrethroid end product. In the case of this activated acid derivative, the unfavourable effect of the DMF liberated in the esterification step can also lead to side reactions, mainly when cyano-substituted pyrethroids at the alpha position of the alcohol are prepared by reacting water, water soluble cyanides and substituted aldehydes (e.g. according to the method of DE-A 2,231,312).

DE-A 13023484 describes a process in which a small amount of DMF is added to a cyclopropane carboxylic acid and thionyl chloride is added to the product, without separation, to form the acid chloride.

The present invention relates to the preparation of optically active and/or racemic cyclopropane carboxylic acid chlorides of the general formula (I) 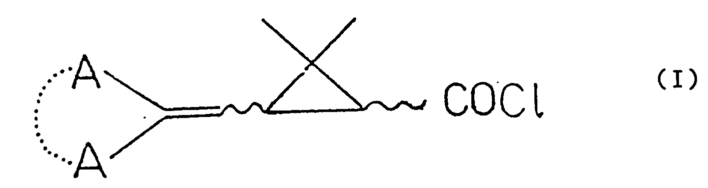
by the reaction of cyclopropane carboxylic acids of the general formula (II) 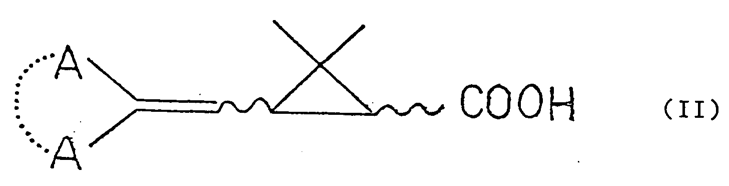
with a complex of the general formula (III) 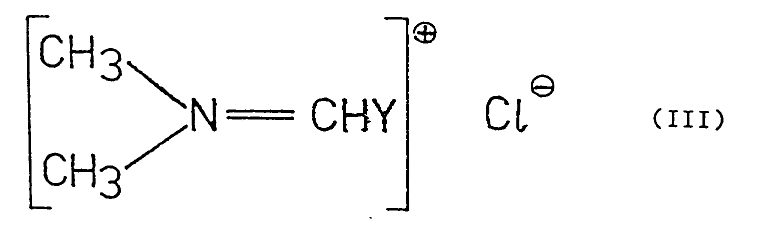
The complex is formed by reacting dimethylformamide with phosgene, thionyl-, phosphoryl-, oxalyl- or a phosphorus chloride at 0-30°C in a molar ratio of 1:1 - 1:0.25, optionally in the presence of an organic aprotic solvent as diluent. The complex (III) obtained, without isolation and optionally in the presence of an apolar aprotic organic solvent as diluent, is reacted at 0-30°C with cyclopropanecarboxylic acid (II) and the emulsion obtained is optionally precipitated. The pure acid halide (I) or its solution in an apolar aprotic solvent is selectively separated from the by-product formed, which is a complex of the general formula (V) 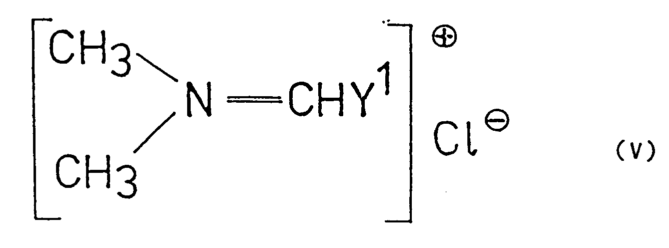
This is removed as a separate liquid phase with impurities dissolved in it, on the basis of the density difference of the phases.

The meanings of the substituents in the formulae are as follows:
- A =: halogen atom or a C₁₋₄ alkyl group,
- Y =: chlorine atom, or a -OSOCl, -OPOCl₂, or a -OPCl₂ group,
- ∼: indicates an α or β-configuration relative to the plane of the cyclopropane ring,
- Y¹=: an -OH, -OSO₂H, -0P0Cl(0H), -OPO(OH)₂, -OPCl(0H), OP(OH)₂ or -OPOCl₂ group.

The present invention is based on the recognition that under suitable conditions, the cyclopropanecarboxylic acid is present at above 0°C to a total extent in the form of a chloride of the general formula (I). When reacting 2,2-dichloro-vinyl-3,3-dimethyl-cyclopropanecarboxylic acid, the characteristic IR peak of the product isolated at 0°C (νC = 0 l779-l781 cm⁻¹) is identical to that of the acid chloride. The infrared spectrum of the acid chloride prepared according to the standard method was quite identical with the spectrum of the product. The NMR spectrum at 0°C also showed only the presence of the acid chloride. The molecular weight calculated from the weight of the sample and its esterification has unanimously given the molecular weight of the acid chloride.

We also recognized that the waste acid formed in the reaction as by-product is complexed immediately with the dimethylformamide present and by choosing the correct quantity of dimethylformamide the waste acid can be totally precipitated and separated.

The contaminating decomposition products formed in small quantity are dissolved in the strongly polar DMF-complex phase and they can be separated together with it. Thus a quite pure acid chloride can be obtained. This is important as a product of 98 w% purity can be safely obtained, and further purification, e.g. by distillation, is superfluous.

When carrying out the reaction with differently substituted cyclopropanecarboxylic acids, no significant difference can be observed regarding the course of the reaction or the possibility of separation. This is surprising, because in the case of other acids the acid halides produced form a homogenous solution with the waste acid complex and thus cannot be separated. This is explained by the surprising apolarity of the acid chloride (I) formed and by the particularly bad solubility and complex forming properties of the same. The present invention is based on the recognition and use of these properties.

We have found it very advantageous to react dimethylformamide in a molar ratio of 1:1-0.9 with phosphoryl chloride or in a molar ratio of 1:1-0.4 with thionyl chloride.

Dimethylformamide can also be used as a diluent. In this case the quantity of the dimethylformamide used as reagent and diluent is expediently at most 4 moles per mole of the starting cyclopropanecarboxylic acid (II).

The increase of the dimethylformamide quantity gradually transforms the emulsion into a homogenous solution. Parallel to this, the quantity and purity of acid chloride to be obtained decreases. Generally, more than the equivalent dimethylformamide quantity can hardly be used, because the dissolvent effect of the dimethylformamide not complexed in both phases becomes effective.

Although the use of a further solvent is generally not needed, the separation of the phases can be improved by the addition of strongly apolar aprotic solvents, which dissolve only the acid halide phase. As diluents one or more C₁₋₁₂ hydrocarbons, optionally halogenated, can be used in a 0.1-5 fold quantity calculated on the weight of the acid halide (I). Such solvents are e.g. hexane, heptane, petrol ether, cyclohexane, carbon tetrachloride etc. An increase of polarity is also disadvantageous.

For reaction with the cyclopropanecarboxylic acid, the complex (III) is used in an amount such that the ratio of the number of halogen atoms of group Y per mole of the cyclopropanecarboxylic acid (II) should be 1-1.5:1.

The most significant advantages of the present process are as follows.

The scale of the reaction can be easily increased it can be carried out in an industrial scale with reagents of industrial quality. The reagents must be totally relieved of water and by using the halogenating agent in a small excess any problem can be solved in a simple way. By using polyvalent halogenating agents, the active halogen content can be exploited fully according to the usual stochiometric proportions of the reagents of this type used for the preparation of conventional acid chlorides. The carboxylic acid activating reaction is very quick and slightly endothermic, and it is expedient to carry it out at room temperature. At a higher temperature of 10-20°C, the instability of the waste acid-complex formed from the reagent and the strongiy acidic medium cause decompositon.

The reaction produces the product in a yield of 95 weight %, and moreover, after the hydrolysis of the waste acid after separation, the remaining 1-5 weight % of the cyclopropanecarboxylic acid can be obtained in a simple way from the hydrolysate and can be recycled. The yield is practically quantitative. The product is generally present, depending on the halogenating agent, in the upper phase and thus after the separation of the waste acid, it can be esterified in the same reaction vessel by the addition of one equivalent of organic amine and the alcohol component solution. Depending on its quality, it can be used immediately in the known one step preparation of pyrethroids which contain a cyano, substituent in the alcohol part (α position) as in e.g. cypermethrin, deltamethrin, cyhalotrin, cyphenotrin, etc.

The reaction is especially favourable for the preparation of optically active acid halides, because these compounds do not suffer any isomerization. Depending on its purity, the product can be stored for years without the smallest sign of decomposition.

The process can be carried out in a simple way even at a scale of several kilomoles. The purity of the product obtained is above 98-99 weight % even at such a scale.

The following examples illustrate the invention.

The purity values in the examples are determined by argentometric methods, potentiometric titration and HPLC measurement after methanolic esterification (Perkin Elmer, column: 250 x 4.6 mm, Spherisorb ODS, 10 µm eluent: 60% methanol 40% aqueous buffer (pH = 2.5), (detector LC-75, (225 nm).

### Example 1

95 g (100 ml, 1.3 moles, 1.3 equivalents) of dimethylformamide are weighed at room temperature into a mixing apparatus and strongly cooled to -10°C. Under intensive stirring and cooling, 160 g (96 ml, 1.04 moles, 1.04 equiv.) of phosphorylchloride are added at a rate such that at the end of the addition the temperature of the mixture is gradually increased to 20°C. The mixture is stirred for 30 minutes, then 210 g (1 mol, l equiv.) of cis-2,2-dimethyl-3-(2',2'-dichlorovinyl)-cyclopropanecarboxylic acid are added. During the addition the temperature of the reaction mixture does not change. At room temperature the mixture is stirred for 2 hours and the emulsion obtained is settled for l hour and the lower phase is run off on crushed ice. The upper colourless phase is separated.

218 g of isomer-free cis-2,2-dimethyl-3-(2',2'-dichlorovinyl)-cyclopropanecarboxylic acid chloride of 99% absolute purity are obtained.
C₈H₉Cl₃0 Mw 227.52

| | | | |
|---|---|---|---|
| Calculated: | C % = 42.23, | H % = 3.98, | Cl % = 46.74; |
| Found: | C % = 42.02, | H % = 3.95, | Cl % = 46.82. |

The isomer purity and active ingredient content are determined by HPLC measurement and by argentometric and potentiometric titration.
Yield: 95 %.
The hydrolyzed waste acid phase is filtered after cooling. The recovered compound is dried. The 8 g of crystalline pale yellow coloured material can be used again in the next reaction after crystallization. Thus the total yield is 99 %.

### Example 2

The process of Example I is used, with the difference that 300 g (1.00 mol) of cis-2,2-dimethyl-3-(2',2-dibromo-vinyl)-cyclopropanecarboxylic acid and 100 ml of n-hexane are added. 368 g of a colourless viscous acid chloride/n-hexane solution is obtained. According to the titrations described above and HPLC measurement, the solution contains 82 % of cis-2,2-dimethyl-3-(2',2'-dibromovinyl)-cyclopropanecarboxylic acid chloride and 18 % of n-hexane. Yield: 95 %. A small portion of the product obtained is evaporated in vacuo and analysed.
C₈H₉Br₂Cl0 Mw: 316.54

| | | | |
|---|---|---|---|
| Calculated: | C % = 30.35, | H % = 2.86, | Cl % = 11.20; |
| Found: | C % = 30.00, | H % = 2.92, | Cl% = 11.28. |

### Example 3

The process of Example l is used, with the difference that 210 g (l mol) of (+)-cis-2,2-dimethyl-3-(2',2'dichlorovinyl)∼cyclopropanecarboxylic acid ([α]²⁵_{D} = 31.3°, c = 1, CHCl₃) is added. Thus 219 g of (+)-cis-2,2-dimethyl-3-(2',2'-dichlorovinyl)-cyclopropanecarboxylic acid chloride of 99 % purity are obtained.
C₈H₉Cl₃0 Mw: 227.52

| | | | |
|---|---|---|---|
| Calculated: | C % = 42.33, | H % = 3.98, | Cl % = 46.74; |
| Found | C % = 42.15, | H % = 4.06, | Cl % = 46.81. |

A small part of the acid chloride is hydrolysed with water and the optical rotation of the obtained (+)-cis-2,2-dimethyl-3-(2',2'dichlorovinyl-cyclopropanecarboxylic acid is measured: [α]²⁵_{D} = 31.3 (c = l, CHCl₃). This shows that is no isomeration occured in the course of the transformation. Yield = 95 %.

### Example 4

The process of Example l is used with the difference that 170 g (1.00 mol) of trans chrysanthemic acid are used. Thus 180 g of trans chrysanthemic acid chloride of 98 % purity are obtained. Yield = 95 %.
C₁₀H₁₅C10 Mw. 186.68.

| | | | |
|---|---|---|---|
| Calculated: | C % = 64.33, | H % = 8.09, | Cl % = 18.99; |
| Found: | C % = 64.28, | H % = 8.06, | Cl % = 19.02. |

### Example 5

Into a mixing apparatus 10 ml of carbon tetrachloride, 170 ml (160.5 g, 2.18 moles, 2.19 equiv.) of dimethylformamide are added and, with strong cooling and intensive stirring, the mixture is cooled to -10°C. At this temperature, 80.2 ml (131.5 g, 1.10 moles, 1.10 equiv.) of thionyl chloride are added. The mixture is stirred for further 30 minutes, whereafter 210 g (l mol, 1 equiv.) of trans-2,2-dimethyl-(2',2'-dichloro-vinyl)-cyclopropanecarboxylic acid are added. After stirring for 2 hours and settling for 2 hours the lower phase is separated. Thus 2.28 g of trans-2,2-dimethyl-(2',2'-dichloro-vinyl)cyclopropanecarboxylic acid chloride are obtained as a colourless oil containing 7 % of carbontetrachloride.
Purity = 93 %.
C₈H₉Cl₃0 Mw. = 227.52
Analysis of an evaporated sample:

| | | | |
|---|---|---|---|
| Calculated: | C % = 42.23, | H % = 3.98, | Cl % = 46.74; |
| Found: | C % = 42.18, | H % = 3.94, | Cl % = 46.92. |

IR (CCl₄) C = 0 1781 cm⁻¹
NMR (COC1₃) (ppm) = 1.30 (d (6H)2CH₃, 2.20, d(1H),
Cl; 2.40 g (1H)C3; 5.60, d(1H); J_{1,3} = 5.2 Hz, J_{CH,3H} = 7.7 Hz.
Yield = 95 %.

### Example 6

Into a mixing apparatus 85 ml (80.25 g, 1.1 moles, 1.1 equiv.) of dimethylformamide are added. According to the method of the previous examples, 131.5 g (80.2 ml, 1.10 moles, 1.1 equiv.) of thionylchloride are added, whereafter the solution is stirred at room temperature in vacuo for 3 hours, while the mixture is transformed to a dense suspension. While vigorously stirring, 210 g (1 mol, 1 equiv.) of cis-2,2-dimethyl-3-(2',2'-dichloro-vinyl)-cyclopropanecarboxylic acid are added and the mixture is reacted at room temperature for 2 hours. After settling the lower phase is separated. 215 g of colourless cis-2,2-dimethyl-3-(2',2'-dichlorovinyl-cyclopropane-carboxylic acid chloride (an oil) are obtained.
Yield = 94 %, purity = 98 %.
Analysis: C₈H₉Cl₃0 Mw. = 227.52

| | | | |
|---|---|---|---|
| Calculated: | C % = 42.23, | H % = 3.98, | Cl % = 46.74, |
| Found: | C % = 42.19, | H % = 4.05, | Cl % = 46.81. |

### Example 7

To a mixture of 85 ml (80.25 g, 1.1 mol, 1.1 equiv.) dimethylformamide and 200 ml of n-haxane at -10°C while vigorously stirring, 140 g (1.10 mol, 1.10 equiv.) of oxalylchloride are added. The suspension is stirred for 30 minutes at -10°C, whereafter at room temperature 210 g (1.0 mol, 1.0 equiv.) of trans-2,2-dimethyl-3-(2',2'-dichloro-vinyl)-cyclopropane-carboxylic acid are added and stirred at room temperature for 3 hours. Meanwhile the suspension gradually becomes transformed into an emulsion. The mixture is settled and the upper phase is separated. 344 g of the colourless trans-2,2-dimethyl-3-(2',2'-dichloro-vinyl)-cyclopropanecarboxylic acid chloride are obtained. The acid chloride content is 61.1%. The IR and NMR spectra of the acid chloride obtained the identical to that of the product of Example 5.
Yield = 93 %.

### Example 8

With vigorous stirring, 20 ml (19 g, 0.26 moles, 2.6 equiv.) of dimethylformamide are cooled to -10°C and 5.9 ml (9.3 g, 6.7 mmoles, 0.67 equiv.) of phosphorus trichloride are added dropwise in a rate such that at the end of addition the temperature of the reaction mixture is 20°C.

The mixture is stirred for 30 minutes, and then 16.8 g (0.1 mol, 0.1 equiv.) of chrysanthemic acid are added. After 2 hours of stirring and 1 hour of settling the upper phase is separated. 18.2 g of colourless chrysanthemic acid chloride are obtained (oil).
Yield: 97.5 %.
Analysis: C₁₀H₁₅Cl0, Mw = 186.68.

| | | | |
|---|---|---|---|
| Calculated: | C % = 64.33, | H % = 8.09, | Cl % = 18.99; |
| Found: | C % = 64.29, | H % = 8.12, | Cl % = 19.05. |

Active ingredient content: 97 %.

### Example 9

To 95 g (100 ml, 1.3 moles, 1:3 equiv.) of dimethylformamide at -10°C, 108.3 g (0.52 moles, 0.52 equiv.) of phosphorus pentachloride are added. The mixture is stirred for 30 minutes at 20°C, whereafter 210 g (1 mol, 1 equiv.) of cis-2,2-dimethyl-3-(2',2'-dichloro-vinyl)-cyclopropanecarboxylic acid are added. The mixture is stirred for 2 hours and the emulsion is settled for 2 hours, the lower phase is allowed to run off on to crushed ice. The upper colourless phase is separated. The product obtained is 215 g of isomer-free cis-2,2-dimethyl-3-(2',2'-dichloro-vinyl)-cyclopropanecarboxylic acid chloride.
Analysis:

| | | | |
|---|---|---|---|
| Calculated: | C % = 42.23, | H % = 3.98, | Cl % = 46.74; |
| Found: | C % = 42.12, | H % = 3.90, | Cl % = 47.05. |

Yield = 93.7 %.

## Claims

1. A process for the preparation of optically active and/or racemic cyclopropane carboxylic acid chlorides of the general formula (I) 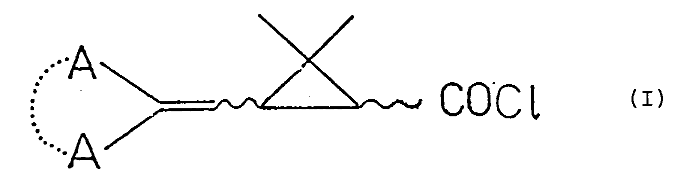 wherein ∼ indicates an α or β-configuration relative to the plane of the cyclopropane ring, and A is a halogen atom or a C₁₋₄ alkyl group,
which comprises forming a complex of the general formula (III) 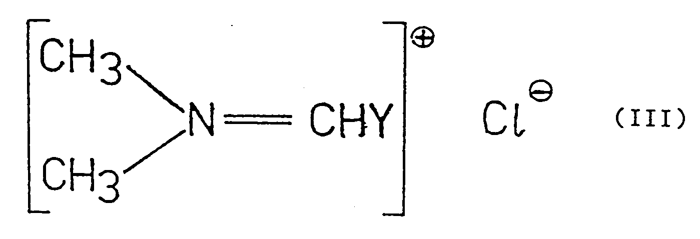 (where Y is a chlorine atom or a -OSOC1, -OPOC1₂ or -0PCl₂ group) by reaction of dimethylformamide at 0-30°C with thionyl, phosphoryl, oxalyl- or a phosphorus chloride or phosgene at a molar ratio of 1:1 - 1:0.25, optionally in the presence of a diluent; thereafter reacting the complex of the formula (III) obtained without isolation (and optionally in the presence of an apolar, aprotic organic solvent as diluent) at a temperature of 0-30°C with a cyclopropanecarboxylic acid of the general formula (II) - 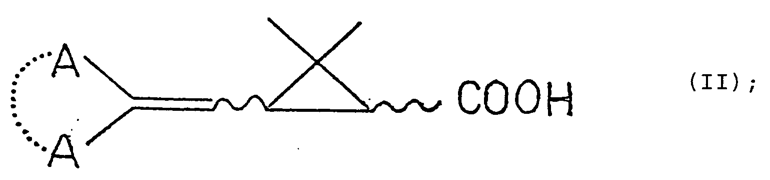 optionally precipitating the emulsion obtained;
and separating on the basis of density difference the pure acid chloride of formula (I) or its solution in an apolar, aprotic solvent from the complex by-product of the general formula (V) 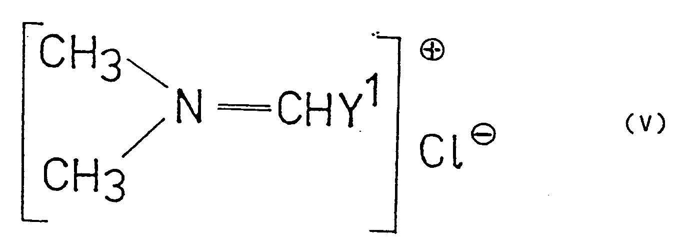 (wherein Y¹ is an -OH, -OSO₂H, -OPOCl(0H), -OPO(OH)₂, -OPCl(OH), -OP(OH)₂ or -OPOCl₂ group), which is in a liquid phase together with dissolved impurities.

2. A process according to claim 1 in which the dimethylformamide is reacted with phosphoryl chloride at a molar ratio of 1:1-0.9 or with thionyl chloride at a molar ratio of 1:1-0.4.

3. A process according to claim 1 in which dimethylformamide is also used as a diluent, and is used in an amount of at most 4 moles per mole of the cyclopropane carboxylic acid (II).

4. A process according to claim 1 or claim 2 in which an organic, aprotic solvent (advantageously an optionally halogenated C₁₋₁₂ hydrocarbon) is used as diluent in a 0.1 - 5 fold amount relative to the weight of the acid chloride (I).

5. A process according to any of claims 1 to 3, in which in the reaction with the cyclopropane carboxylic acid (II), the complex (III) is used in an amount such that the ratio of the number of halogen atoms in the Y group per mole of the acid (II) is 1-1.5:1.

## Patentansprüche

1. Verfahren zur Herstellung optisch aktiver und/oder racemischer Cyclopropancarbonsäurechloride der allgemeinen Formel (I) 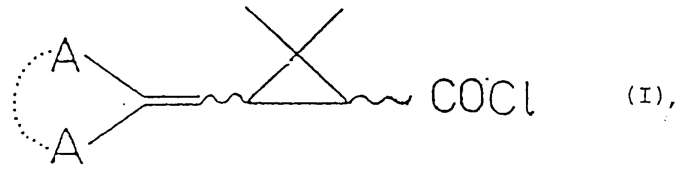 worin bedeuten:
die Wellenlinie 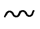 α-oder β-Konfiguration in Bezug auf die Ebene des Cyclopropanrings,
und
A ein Halogenatom oder C₁₋₄-Alkyl,
das folgende Schritte umfaßt:
Herstellung eines Komplexes der allgemeinen Formel (III) 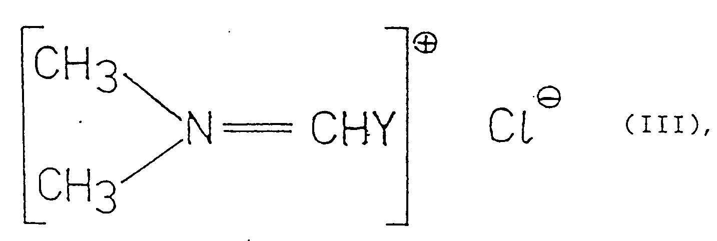 in der Y Chlor, -OSOCl, -OPOCl₂ oder -OPCl₂ bedeutet, durch Umsetzung von Dimethylformamid mit Thionylchlorid, Phosphorylchlorid, Oxalylchlorid, einem Phosphorchlorid oder Phosgen bei 0 bis 30 °C und einem Molverhältnis von 1:1 bis 1:0,25,
ggf. in Gegenwart eines Verdünnungsmittels,
anschließend Umsetzung des erhaltenen Komplexes der Formel (III) ohne Isolierung, ggf. in Gegenwart eines apolaren, aprotischen organischen Lösungsmittels als Verdünnungsmittel, bei einer Temperatur von 0 bis 30 °C mit einer Cyclopropancarbonsäure der allgemeinen Formel (II) 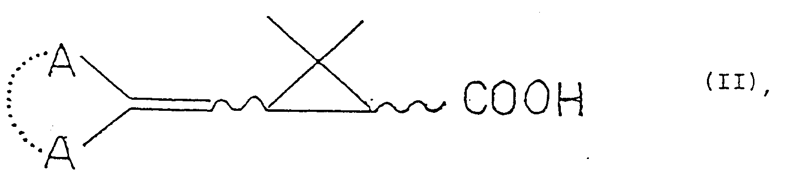 ggf. Phasentrennung der erhaltenen Emulsion,
und
Abtrennung des reinen Säurechlorids der Formel (I) oder seiner Lösung in einem apolaren, aprotischen Lösungsmittel vom Komplex-Nebenprodukt der allgemeinen Formel (V) 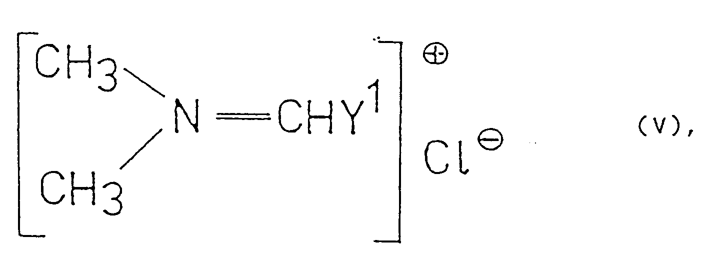 worin Y¹ -OH, -OSO₂H, -OPOCl(OH), -OPO(OH)₂, -OPCl(OH), -OP(OH)₂ oder OPOCl₂ bedeutet,
das in einer flüssigen Phase zusammen mit gelösten Verunreinigungen vorliegt, auf der Basis des Dichteunterschieds.

2. Verfahren nach Anspruch 1, wobei Dimethylformamid mit Phosphorylchlorid bei einem Molverhältnis von 1:1 bis 0,9 oder mit Thionylchlorid bei einem Molverhältnis von 1:1 bis 0,4 umgesetzt wird.

3. Verfahren nach Anspruch 1, wobei Dimethylformamid auch als Verdünnungsmittel verwendet und in einer Menge von mindestens 4 mol pro Mol Cyclopropancarbonsäure (II) eingesetzt wird.

4. Verfahren nach Anspruch 1 oder 2, wobei ein organisches, aprotisches Lösungsmittel, vorteilhaft ein ggf. halogenierter C₁₋₁₂-Kohlenwasserstoff, als Verdünnungsmittel in der 0,1- bis 5-fachen Menge, bezogen auf das Gewicht des Säurechlorids (I), eingesetzt wird.

5. Verfahren nach einem der Ansprüche 1 bis 3, wobei der Komplex (III) bei der Umsetzung mit der Cyclopropancarbonsäure (II) in einer solchen Menge eingesetzt wird, daß das Verhältnis der Anzahl der Halogenatome in der Gruppe Y pro Mol Säure (II) 1 bis 1,5:1 beträgt.

## Revendications

1. Procédé de préparation de chlorures d'acide cyclopropanecarboxylique optiquement actifs et/ou racémiques de la formule générale (II) 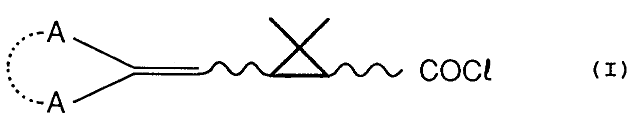 où - indique une configuration α ou β par rapport au plan du noyau cyclopropane et A est un atome d'halogène ou un groupe alkyle C₁₋₄,
lequel comprend la formation d'un complexe de la formule générale (III) 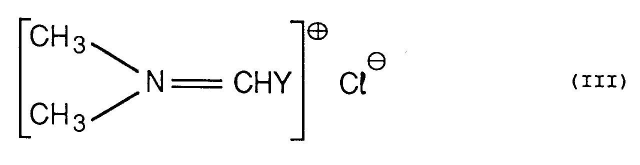 (où Y est un atome de chlore ou un groupe -OSOCl, -OPOCl₂ ou -OPCl₂) obtenu par réaction de diméthylformamide à 0-30°C avec un chlorure de thionyle, de phosphoryle, d'oxalyle ou un chlorure de phosphore ou un phosgène selon un rapport molaire de 1 : 1 - 1 : 0,25, optionnellement en présence d'un diluant ;
puis la réaction du complexe de la formule (III) obtenu sans isolement (et optionnellement en présence d'un solvant organique apolaire aprotique en tant que diluant) à une température de 0-30°C avec un acide cyclopropanecarboxylique de la formule générale (II) 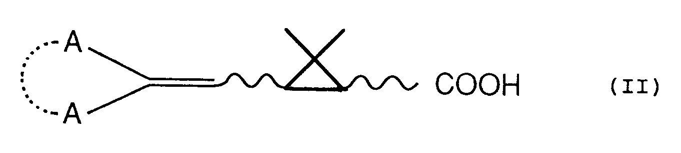 et optionnellement, la précipitation de l'émulsion obtenue ;
et la séparation sur la base de la différence de densité du chlorure d'acide pur de la formule (I) ou de sa solution dans un solvant apolaire aprotique à partir du produit secondaire complexe de la formule générale (V) 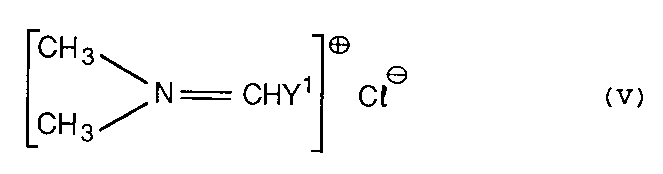 (où Y¹ est un groupe -OH, -OSO₂H, -OPOCl(OH), -OPO(OH)₂, -OPCl(OH), -OP(OH)₂ ou -OPOCl₂) qui est dans une phase liquide ensemble avec des impuretés dissoutes.

2. Procédé selon la revendication 1, dans lequel du diméthylformamide est amené à réagir avec du chlorure de phosphoryle selon un rapport molaire de 1 : 1-0,9 ou avec du chlorure de thionyle selon un rapport molaire de 1 : 1-0,4.

3. Procédé selon la revendication 1, dans lequel du diméthylformamide est également utilisé en tant que diluant et est utilisé selon une quantité d'au moins 4 moles par mole d'acide cyclopropanecarboxylique (II).

4. Procédé selon la revendication 1 ou 2, dans lequel un solvant organique aprotique (avantageusement un hydrocarbure C₁₋₁₂ optionnellement halogéné) est utilisé en tant que diluant selon une quantité qui vaut de 0,1 à 5 fois le poids du chlorure d'acide (I).

5. Procédé selon l'une quelconque des revendications 1 à 3, dans lequel, lors de la réaction avec l'acide cyclopropanecarboxylique (II), le complexe (III) est utilisé selon une quantité qui est telle que le rapport du nombre d'atomes d'halogène dans le groupe Y par mole de l'acide (II) vaut 1-1,5 : 1.
